# EUROPÄISCHE PATENTSCHRIFT

(11) **EP 4 135 607 B1**
(45) Veröffentlichungstag und Bekanntmachung des Hinweises auf die Patenterteilung: **28.01.2026**
(21) Anmeldenummer: 21720405.6
(22) Anmeldetag: 15.04.2021
(51) Int. Cl.: A61B 17/70

(54) **CHIRURGISCHES FIXATIONSSYSTEM**
SURGICAL FIXATION SYSTEM
SYSTÈME DE FIXATION CHIRURGICAL

(30) Priorität: 17.04.2020 DE 102020110516
(43) Veröffentlichungstag der Anmeldung: 22.02.2023
(73) Patentinhaber: Aesculap AG, 78532 Tuttlingen (DE)
(72) Erfinder: KRÜGER, Sven, 78647 Trossingen (DE); WEIß, Josef-Benedikt, 78628 Rottweil (DE); TAN, Jian-Zoing, 78532 Tuttlingen (DE)
(74) Vertreter: Hoeger, Stellrecht & Partner Patentanwälte mbB
(86) Internationale Anmeldenummer: PCT/EP2021/059735
(87) Internationale Veröffentlichungsnummer: WO 2021/209531

(56) Entgegenhaltungen:
- EP-A1- 3 174 482
- US-A1- 2007 233 078
- US-A1- 2012 253 408
- US-A1- 2014 180 346

## Beschreibung

Die vorliegende Erfindung betrifft ein chirurgisches Fixationssystem, umfassend mindestens ein Verankerungselement mit einem Verankerungsabschnitt zum Verankern an einem Knochen und mit einem Aufnahmeabschnitt für ein Stabilisierungselement zur Verbindung mit einem weiteren Verankerungselement, wobei das Stabilisierungselement im Aufnahmeabschnitt anordenbar und darin mittels eines Fixierelementes fixierbar ist, wobei das Fixationssystem ein am Aufnahmeabschnitt angeordnetes und am Verankerungsabschnitt anliegendes Anlageelement zum Anlegen des Stabilisierungselementes umfasst, wobei das Anlageelement mindestens einen Verformungsbereich zum Verformen abhängig von einer das Stabilisierungselement beaufschlagenden Fixierkraft des Fixierelementes aufweist, wobei das Anlageelement zumindest abschnittsweise hülsenförmig ausgestaltet ist und an einer Stirnseite einen Anlagebereich für das Stabilisierungselement aufweist, wobei mindestens ein Verformungsbereich unterhalb des Anlagebereiches des Anlageelementes für das Stabilisierungselement angeordnet ist.

Ein derartiges Fixationssystem, umfassend mindestens ein Verankerungselement mit einem Verankerungsabschnitt zum Verankern an einem Knochen und mit einem Aufnahmeabschnitt für ein Stabilisierungselement zur Verbindung mit einem weiteren Verankerungselement, wobei das Stabilisierungselement im Aufnahmeabschnitt anordenbar und darin mittels eines Fixierelementes fixierbar ist, kommt zum Beispiel bei der Behandlung von Frakturen im Wirbelsäulenbereich zum Einsatz. Hierbei können Verankerungselemente vorgesehen sein, zum Beispiel Knochenschrauben, insbesondere Pedikelschrauben. Ein beispielsweise stabförmiges Stabilisierungselement kann in den jeweiligen Aufnahmeabschnitt der Knochenschraube eingesetzt und darin zum Beispiel mittels eines Schraubelementes klemmend fixiert werden. Der Aufnahmeabschnitt kann in der Praxis beispielsweise zwei im Abstand zueinander angeordnete Segmente aufweisen, zwischen denen eine Öffnung für das Stabilisierungselement angeordnet ist. Die Segmente können zum Beispiel ein Innengewinde zum Verschrauben mit einem Außengewinde des Schraubelementes aufweisen.

Je nach Behandlung kann es wünschenswert oder erforderlich sein, zum Beispiel Stabilisierungselemente unterschiedlicher Beschaffenheit am Verankerungselement zu adaptieren. Beispielsweise können die Stabilisierungselemente unterschiedliche Materialien und/oder, im Fall von Stabelementen, unterschiedliche Durchmesser aufweisen.

In der US 2005/0277928 A1 ist ein Fixationssystem beschrieben, bei dem zur Anpassung des Verankerungselementes an unterschiedliche Stabdurchmesser ein Aufnahmeabschnitt mit einem im Wesentlichen U-förmigen Anlageelement vorgesehen ist. Zwar können Stabelemente unterschiedlichen Durchmessers in diesem Fall besser als im Vergleich zu herkömmlichen Fixationssystemen adaptiert werden. Jedoch führt das U-förmige Anlageelement zu unterschiedlichen Abständen des Stabelementes relativ zum Verankerungselement. Dies ist für die Behandlung unerwünscht. Die Beschaffenheit des Anlageelementes führt ferner zu Linien- oder Punktauflagen des Stabelementes mit der Gefahr einer instabilen Fixierung im Aufnahmeabschnitt. Linien- und Punktauflagen können aufgrund hoher Fixierkräfte mit dem Fixierelement zu Lockerungseffekten und/oder Korrosionseffekten führen. Das Fixieren von gebogenen Stabelementen ist häufig klinisch erforderlich, es kann jedoch zu einer ungünstigen Anlage des Stabelementes am Anlageelement führen. Als Folge können sich Zwangskräfte, Lockerung, Bruch und Korrosion ergeben.

Die US 2005/0277928 A1 beschreibt ferner, dass am Anlageelement Schenkel angeordnet sein können, die zum Einführen des Stabilisierungselementes relativ zueinander gespreizt und nach dem Einführen einander wieder angenähert werden können. Dies erlaubt es, das Stabilisierungselement vorläufig im Aufnahmeabschnitt zu fixieren.

Die US 2012/253408 A1 offenbart eine Schraubenanordnung mit verformbarer Buchse. Aus der US 2007/233078 A1sind verschwenkbare Gelenke mit einem vorgegebenen Bewegungswiderstand für Wirbelsäulenimplantate bekannt sowie ein Verfahren zur Verwendung derartiger Gelenke. Die US 2014/180346 A1 betrifft Vorrichtungen und Verfahren für die dynamische Befestigung von Skelettstrukturen. Eine Polyaxialschraube für chirurgische Implantate ist in der EP 3 174 482 A1 beschrieben.

Aufgabe der vorliegenden Erfindung ist es, ein gattungsgemäßes Fixationssystem bereitzustellen, das vielseitiger einsetzbar ist.

Diese Aufgabe wird bei einem Fixationssystem der eingangs genannten Art erfindungsgemäß dadurch gelöst, dass der mindestens eine Verformungsbereich durch mindestens eine Materialausnehmung am Anlageelement gebildet ist oder eine solche umfasst.

Beim erfindungsgemäßen Fixationssystem besteht die Möglichkeit, das Stabilisierungselement über das Fixierelement mit einer Fixierkraft, insbesondere einer Klemmkraft, am Aufnahmeabschnitt zu fixieren. Vorgesehen ist ein Anlageelement am Aufnahmeabschnitt, das infolge der Fixierkraft an mindestens einem Verformungsbereich verformt werden kann. Vorzugsweise kann eine definierte Verformung des Anlageelementes an dem mindestens einen Verformungsbereich erzielt werden. Dies erlaubt es, unterschiedliche Stabilisierungselemente, die sich beispielsweise hinsichtlich ihres Materials und/oder ihrer Geometrie (beispielsweise Durchmesser) voneinander unterscheiden, besser am Aufnahmeabschnitt zu adaptieren. Zugleich liegt das Anlageelement am Verankerungsabschnitt an, so dass vorzugsweise ein verbesserter Sitz des Stabilisierungselementes am Verankerungselement erzielt werden kann. Vorzugsweise kann ein Verankerungselement dabei mit einer Vielzahl unterschiedlicher Stabilisierungselemente zum Einsatz kommen, wobei vorzugsweise ein identischer Abstand zum Verankerungselement erzielt werden kann. Das Fixationssystem weist dadurch eine höhere Vielseitigkeit auf. Das Vorhalten von Fixationssystemen für unterschiedliche Behandlungen wird erheblich vereinfacht. Vorzugsweise kann infolge der Verformung des Anlageelementes eine Vergleichmäßigung der auf das Stabilisierungselement wirkenden Kräfte erzielt werden und/oder eine flächige Anlage am Anlageelement bei insbesondere zuverlässigem Sitz am Verankerungselement. Dies erlaubt eine zuverlässigere Fixierung des Stabilisierungselementes als bei herkömmlichen Fixationssystemen. Gemäß der Erfindung ist das Anlageelement zumindest abschnittsweise hülsenförmig ausgestaltet, wobei es an einer Stirnseite einen Anlagebereich für das Stabilisierungselement aufweist. Die Stirnseite kann beispielsweise einer weiteren Stirnseite gegenüberliegen, über die das Anlageelement am Verankerungsabschnitt anliegen kann. Ferner ist gemäß der Erfindung mindestens ein Verformungsbereich unterhalb eines Anlagebereiches des Anlageelementes für das Stabilisierungselement angeordnet ist. Die Kraft des Fixierelementes kann insbesondere über das Stabilisierungselement auf den Anlagebereich, insbesondere Auflagebereich, oder den darunterliegenden mindestens einen Verformungsbereich des Anlageelementes gerichtet sein. Überdies ist gemäß der Erfindung der mindestens eine Verformungsbereich durch mindestens eine Materialausnehmung am Anlageelement gebildet ist oder eine solche umfasst.

Bei einer bevorzugten Ausführungsform der Erfindung kann vorgesehen sein, dass das Anlageelement getrennt vom Aufnahmeabschnitt gebildet und in dem Aufnahmeabschnitt angeordnet ist. Dies erhöht die Vielseitigkeit des Fixationssystems. Beispielsweise können unterschiedliche Anlageelemente vorgesehen sein, die abhängig von der durchzuführenden Behandlung wahlweise in dem Aufnahmeabschnitt positioniert werden können. Insbesondere besteht hierbei die Möglichkeit eines modularen Aufbaus des Fixationssystems. Durch separate Fertigung des Aufnahmeabschnittes und des mindestens einen Anlageelementes können die jeweiligen Vorteile dieser beiden Komponenten im Hinblick auf eine bestmögliche Versorgung herausgestellt werden.

Vorgesehen sein kann bei einer andersartigen vorteilhaften Ausführungsform, dass der Aufnahmeabschnitt das Anlageelement umfasst oder ausbildet. Dies erlaubt zum Beispiel eine konstruktiv einfache Fertigung.

Das Anlageelement ist infolge der Fixierkraft des Fixierelementes insbesondere knautschbar.

Vorgesehen sein kann, dass das Anlageelement zumindest am mindestens einen Verformungsbereich elastisch verformbar ausgebildet ist.

Vorgesehen sein kann, dass das Anlageelement zumindest am mindestens einen Verformungsbereich plastisch verformbar ausgebildet ist.

Das Anlageelement kann bei einer bevorzugten Ausführungsform der Erfindung einstückig sein.

Als vorteilhaft kann es sich erweisen, wenn mindestens ein Verformungsbereich an einem Anlagebereich des Anlageelementes für das Stabilisierungselement angeordnet ist. Die Kraft des Fixierelementes kann insbesondere über das Stabilisierungselement auf den Anlagebereich, insbesondere Auflagebereich, oder den darunterliegenden mindestens einen Verformungsbereich des Anlageelementes gerichtet sein.

Alternativ oder ergänzend kann mindestens ein Verformungsbereich an oder seitlich neben einem seitlichen Anlagebereich für das Stabilisierungselement angeordnet sein. Beispielsweise weist das Anlageelement seitliche Stützglieder für das Stabilisierungselement auf, die jeweils einen Anlagebereich umfassen. Durch die Verformung des Stützgliedes kann eine Anpassung des Anlageelementes an das Stabilisierungselement zur Seite erfolgen.

Bei einer bevorzugten Ausführungsform der Erfindung kann vorgesehen sein, dass das Anlageelement einen dem Stabilisierungselement zugewandten ersten Anlageelementabschnitt und einen dem Verankerungsabschnitt zugewandten zweiten Anlageelementabschnitt umfasst. Günstig kann es insbesondere sein, wenn der erste Anlageelementabschnitt den mindestens einen Verformungsbereich umfasst oder ausbildet und, zumindest abschnittsweise, eine höhere Verformbarkeit infolge der Fixierkraft aufweist als der zweite Anlageelementabschnitt. Über den ersten Anlageelementabschnitt kann wie erwähnt eine Anpassung an das Stabilisierungselement und vorzugsweise eine Mehrzahl von Stabilisierungselementen erfolgen. Hierbei kann sich der mindestens eine Verformungsbereich abhängig von der Fixierkraft an Größe und/oder Form des Stabilisierungselementes verformen und das Anlageelement dadurch an das Stabilisierungselement anpassen. Am zweiten Anlageelementabschnitt weist das Anlageelement eine geringere Verformbarkeit auf als am ersten Anlageelementabschnitt. Hierunter kann vorliegend insbesondere verstanden werden, dass das Anlageelementabschnitt zweiten Anlageelementabschnitt "härter" ist als am ersten Anlageelementabschnitt. Am Verankerungsabschnitt kann dadurch zuverlässiger Sitz, vorzugsweise durch Kraftschluss und/oder Formschluss, erzielt werden.

Die Anlageelementabschnitte können beispielsweise aus unterschiedlichen Materialien gefertigt sein oder, bei einer Fertigung aus dem identischen Material, eine unterschiedliche Beschaffenheit hinsichtlich der Verformbarkeit aufweisen.

Die Anlageelementabschnitte können beispielsweise getrennt voneinander gebildet und aneinandergefügt sein. Alternativ kann vorgesehen sein, dass die Anlageelementabschnitte miteinander insbesondere einstückig gebildet sind.

Beispielsweise ist eine hinsichtlich der Verformbarkeit des Anlageelementes diskrete Abschnittsgrenze zwischen dem ersten Anlageelementabschnitt und dem zweiten Anlageelementabschnitt vorgesehen. Hierbei kann sich beispielsweise eine stufenweise Änderung der Verformbarkeit am Anlageelementabschnitt geben.

Die Abschnittsgrenze ist zum Beispiel quer und insbesondere senkrecht zu einer Fixierrichtung des Fixierelementes in Richtung des Verankerungsabschnittes ausgerichtet.

Vorgesehen sein kann bei einer bevorzugten Ausführungsform, dass hinsichtlich der Verformbarkeit des Anlageelementes ein Übergangsabschnitt vorhanden ist, über den der erste Anlageelementabschnitt und der zweite Anlageelementabschnitt ineinander übergehen.

Der mindestens eine Verformungsbereich kann bei einer bevorzugten Ausführungsform eine Erstreckung parallel zu einem Anlagebereich des Anlageelementes für das Stabilisierungselement aufweisen.

Vorgesehen sein kann, dass der mindestens eine Verformungsbereich symmetrisch am Anlageelement angeordnet oder gebildet ist bezüglich einer eine Achse des Aufnahmeabschnittes enthaltenden Symmetrieebene. Insbesondere kann hierbei das Anlageelement koaxial zum Aufnahmeabschnitt positioniert sein. Die Symmetrieebene ist beispielsweise eine Mittelebene des Aufnahmeabschnittes. Durch eine symmetrische Anordnung des Verformungsbereiches kann vorteilhafterweise eine Vergleichmäßigung der Fixierkräfte erzielt werden.

Die Materialausnehmung ist beispielsweise eine Ausnehmung an einer Oberfläche des Anlageelementes, wobei das Anlageelement am Rand der Ausnehmung verformbar ist. Hierbei kann vorteilhafterweise vorgesehen sein, dass das Stabilisierungselement formschlüssig in die Ausnehmung eingreift.

Bei einer bevorzugten Ausführungsform der Erfindung ist oder umfasst die Materialausnehmung eine Durchgangsöffnung des Anlageelementes. Durch Bildung einer und beispielsweise mehrerer Durchgangsöffnungen kann eine konstruktiv einfache Umsetzung der Erfindung erfolgen. Beispielsweise wird das Anlageelement unter der Wirkung der Fixierkraft geknautscht, wobei die Durchgangsöffnungen formveränderlich sind.

Die Durchgangsöffnung kann beispielsweise einen kreisrunden, elliptischen, ovalen, runden, langlochförmigen oder einen unrunden Querschnitt aufweisen. "Rund" kann vorliegend insbesondere als nicht-eckig verstanden werden.

Vorgesehen sein kann, dass die Materialausnehmung eine Erstreckung entlang des im Aufnahmeabschnitt angeordneten Stabilisierungselementes aufweist. Das Stabilisierungselement kann infolge der Ausgestaltung des Aufnahmeabschnittes, insbesondere mit zwei im Abstand zueinander angeordneten Segmenten, eine Vorzugsrichtung aufweisen. Die Materialausnehmung kann entlang insbesondere dieser Vorzugsrichtung erstreckt sein. Dies erlaubt eine verbesserte Anpassung des Anlageelementes an die Geometrie des Stabilisierungselementes im Hinblick auf eine möglichst vorteilhafte Verformung.

Vorgesehen sein kann, dass die Materialausnehmung eine Erstreckung radial zu einer vom Anlageelement definierten Achse aufweist.

Bei einer bevorzugten Ausführungsform der Erfindung ist die Materialausnehmung ein im Aufnahmeelement gebildeter, allseits umschlossener Hohlraum.

Vorteilhaft kann es sein, wenn das Anlageelement zwei oder mehr Verformungsabschnitte umfasst, die hinsichtlich ihrer Verformbarkeit unterschiedliche Materialen umfassen oder aus unterschiedlichen Materialien gefertigt sind, wobei der mindestens eine Verformungsbereich aufgrund der materialmäßig unterschiedlichen Beschaffenheit der zwei oder mehr Verformungsabschnitte gebildet ist.

Beispielsweise grenzen zwei Verformungsabschnitte mit unterschiedlicher Verformbarkeit aneinander. Infolge der Kraftbeaufschlagung kann sich das Anlageelement an dem weicheren Verformungsabschnitt und/oder im Übergangsabschnitt zwischen dem weicheren und dem weniger weichen Verformungsabschnitt verformen.

Die zwei oder mehr Verformungsabschnitte können getrennt voneinander gebildete und aneinandergefügte Verformungsabschnitte des Anlageelementes sein.

Alternativ kann vorgesehen sein, dass die zwei oder mehr Verformungsabschnitt einstückig miteinander gebildet sind.

Vorteilhaft kann es sein, wenn ein erster Verformungsabschnitt vorgesehen ist, der zumindest teilweise von mindestens einem zweiten Verformungsabschnitt umgeben ist, wobei die Verformbarkeit des ersten Verformungsabschnittes größer ist als die Verformbarkeit des mindestens einen zweiten Verformungsabschnittes. Insbesondere kann der zweite Verformungsabschnitt den ersten Verformungsabschnitt vollständig umgeben.

Es können bei einer bevorzugten Ausführungsform drei oder mehr Verformungsabschnitte vorgesehen sein, wobei die Verformbarkeit eines jeweiligen Verformungsabschnitte, der einen weiteren Verformungsabschnitt zumindest teilweise umgibt, geringer ist als die Verformbarkeit des umgebenen Verformungsabschnittes.

Es kann vorgesehen sein, dass die Verformungsabschnitte unterschiedlicher Verformbarkeit unmittelbar aneinandergrenzen, so dass sich eine diskrete, stufenweise Änderung der Verformbarkeit am Anlageelement ergibt.

Alternativ kann vorgesehen sein, dass ein Übergangsabschnitt zwischen den Verformungsabschnitten unterschiedlicher Verformbarkeit vorhanden ist, so dass sich die Verformbarkeit schrittweise und insbesondere kontinuierlich ändert.

Günstig kann es sein, wenn das Anlageelement eine Mehrzahl von Verformungsbereichen umfasst, insbesondere mit einer Mehrzahl von Materialausnehmungen.

Hierbei kann insbesondere vorgesehen sein, dass zwei oder mehr identisch ausgestaltete Verformungsbereiche vorgesehen sind.

Alternativ oder ergänzend können zwei oder mehr unterschiedlich ausgestaltete Verformungsbereiche vorgesehen sein.

Zwei oder mehr Durchgangsöffnungen am Anlageelement können zum Beispiel parallel zueinander angeordnet und ausgerichtet sein.

Vorgesehen sein kann, dass mehrere Durchgangsöffnungen "auf Lücke" zueinander angeordnet sind.

Als günstig kann es sich erweisen, wenn zwei Verformungsbereiche vorgesehen sind, die im Abstand zueinander und miteinander fluchtend am Anlageelement angeordnet sind.

Beispielsweise weist das Anlageelement, zum Beispiel bei einer hülsenförmigen Ausgestaltung, zwei einander diametral gegenüberliegende Verformungsbereiche auf. Die Verformungsbereiche, zum Beispiel gebildet durch Durchgangsöffnungen oder Verformungsabschnitte unterschiedlicher Verformbarkeit, fluchten vorzugsweise miteinander. Die Richtung der Flucht entspricht vorteilhafterweise der Erstreckungsrichtung des Stabilisierungselementes im Aufnahmeabschnitt.

Beispielsweise kann der vorstehend erwähnte erste Anlageelementabschnitt an einer Stirnseite den Anlagebereich umfassen oder bilden.

Das Anlageelement kann bei einer bevorzugten Ausführungsform zwei im Abstand zueinander angeordnete Stützglieder umfassen, die eine sich verjüngende Vertiefung seitlich begrenzen, wobei das Stabilisierungselement zwischen den Stützgliedern in der Vertiefung positionierbar ist. Durch die Stützglieder kann das Stabilisierungselement seitlich abgestützt und dadurch besonders vorteilhaft im Aufnahmeabschnitt fixiert werden.

Vorzugsweise ist zumindest an einem Stützglied, bevorzugt an beiden Stützgliedern, ein Verformungsbereich angeordnet.

Das Anlageelement kann vorzugsweise koaxial zum Aufnahmeabschnitt und/oder zum Verankerungsabschnitt ausgerichtet oder ausrichtbar sein.

Günstig ist es, wenn der Verankerungsabschnitt und das Anlageelement aneinander angepasste, insbesondere zumindest abschnittsweise kugelschalenförmige Anlagebereiche umfassen. Beispielsweise ist das Verankerungselement eine Polyaxialschraube mit einem kugelförmigen Verankerungsabschnitt. Das diesbezüglich angepasste Anlageelement kann dadurch vorzugsweise formschlüssig am Anlagebereich des Verankerungsabschnittes anliegen und eine definierte Position relativ zum Verankerungsabschnitt einnehmen.

Beispielsweise kann der vorstehend erwähnte zweite Anlageelementabschnitt an einer Stirnseite den Anlagebereich umfassen oder bilden. Über den Anlagebereich kann ein zuverlässiger Sitz des Anlageelementes mit vorzugsweise optimierter Passung am Verankerungsabschnitt erzielt werden.

Günstigerweise ist das Anlageelement formschlüssig im Aufnahmeabschnitt angeordnet. Beispielsweise ist das Anlageelement formschlüssig zwischen zwei im Abstand zueinander angeordneten Segmenten des Aufnahmeabschnitts positioniert.

Das Fixationssystem kann beispielsweise zwei oder mehr Verankerungselemente umfassen. Die Verankerungselemente können vorzugsweise identisch ausgestaltet sein.

Das Fixationssystem umfasst bevorzugt zwei oder mehr Anlageelemente. Hierbei können mindestens zwei identisch ausgestaltete und/oder mindestens zwei unterschiedlich ausgestaltete Anlageelemente vorgesehen sein.

Das Fixationssystem umfasst vorzugsweise mindestens ein Stabilisierungselement, insbesondere eine Mehrzahl von Stabilisierungselementen. Mindestens zwei Stabilisierungselemente können identisch ausgestaltet sein. Alternativ oder ergänzend sind mindestens zwei Stabilisierungselemente unterschiedlich ausgestaltet.

Das Fixationssystem umfasst bevorzugt mindestens ein Fixierelement, insbesondere eine Mehrzahl von Fixierelementen. Mindestens zwei Fixierelemente können identisch ausgestaltet sein. Alternativ oder ergänzend sind mindestens zwei Fixierelemente unterschiedlich ausgestaltet.

Das mindestens eine Verankerungselement ist beispielsweise eine Knochenschraube. Die Knochenschraube kann eine Monoaxialschraube sein. Alternativ kann die Knochenschraube eine Polyaxialschraube sein, bei der der Aufnahmeabschnitt relativ zum Verankerungsabschnitt schwenkbar ist.

Das mindestens eine Stabilisierungselement ist bevorzugt ein Stabelement.

Das mindestens eine Fixierelement ist vorzugsweise ein mit dem Aufnahmeabschnitt verschraubbares Schraubelement.

Als Materialien für das Anlageelement kommen beispielsweise Ti₆Al₄V, Titan, PEEK oder eine CoCr-Legierung zum Einsatz. Kombinationen der voranstehenden Materialien sind denkbar.

Das Anlageelement weist insbesondere am Verformungsbereich beispielsweise ein Elastizitätsmodul von ungefähr 50.000 bis 150.000 MPa, beispielsweise ungefähr 100.000 bis 120.000 MPa für Ti₆Al₄V auf. Das Elastizitätsmodul kann beispielsweise ungefähr 2.000 bis 6.000 MPa (beispielsweise 3.000 bis 4.000 MPa) für Peek betragen und zum Beispiel ungefähr 200.000 bis 300.000 MPa für CoCr, vorzugsweise ungefähr 230.000 bis 270.000 MPa.

Eine auf das Stabilisierungselement wirkende Fixierkraft des Fixierelementes kann ungefähr 3 kN bis ungefähr 7 kN bei Wirbelsäulen-Fixationssystemen im Lumbalbereich und ungefähr 0,8 kN bis 2,5 kN für ein Wirbelsäulen-Fixationssystem im Zervikalbereich betragen.

Demgegenüber sind die Kräfte auf das Stabilisierungselement im implantierten Zustand üblicherweise deutlich geringer. Beispielsweise treten Axialkräfte bis zu ungefähr 300 N auf das Stabilisierungselement auf. Ein Biegemoment kann beispielsweise bis ungefähr ca. 8 Nm betragen.

Eine Deformation des Anlageelementes gemäß der Erfindung kann beispielsweise ungefähr im Bereich von 0,1 mm bis 3 mm liegen, vorzugsweise zwischen 0,2 mm und 1,5 mm.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine schematische perspektivische Ansicht eines erfindungsgemäßen Fixationssystems zum Verbinden von zwei Wirbeln miteinander;
- Figur 2:: eine perspektivische Darstellung eines Anlageelementes des Fixationssystems aus Figur 1;
- Figur 3:: eine Schnittansicht des Fixationssystems aus Figur 1 in einer Teildarstellung, wobei ein Stabilisierungselement an dem Anlageelement gemäß Figur 2 anliegt und von einem Fixierelement noch nicht mit einer Fixierkraft beaufschlagt ist;
- Figur 4:: eine Darstellung entsprechend Figur 3, wobei das Stabilisierungselement mittels des Fixierelementes mit einer Fixierkraft beaufschlagt und dadurch klemmend fixiert ist;
- Figuren 5 und 6:: Teildarstellungen entsprechend den Figuren 3 und 4, wobei ein andersartiges Anlageelement zum Einsatz kommt;
- Figuren 7 und 8:: Teildarstellungen entsprechend den Figuren 3 und 4, wobei ein andersartiges Anlageelement zum Einsatz kommt;
- Figuren 9 und 10:: Teildarstellungen entsprechend den Figuren 3 und 4, wobei ein andersartiges Anlageelement zum Einsatz kommt;
- Figur 11:: eine perspektivische Darstellung eines andersartigen Anlageelementes für das Stabilisierungselement;
- Figuren 12 und 13:: Teildarstellungen entsprechend den Figuren 3 und 4, wobei das Anlageelement aus Figur 11 zum Einsatz kommt;
- Figuren 14 bis 18:: eine jeweilige Teildarstellung entsprechend Figur 3, wobei jeweils andersartige Anlageelemente zum Einsatz kommen.

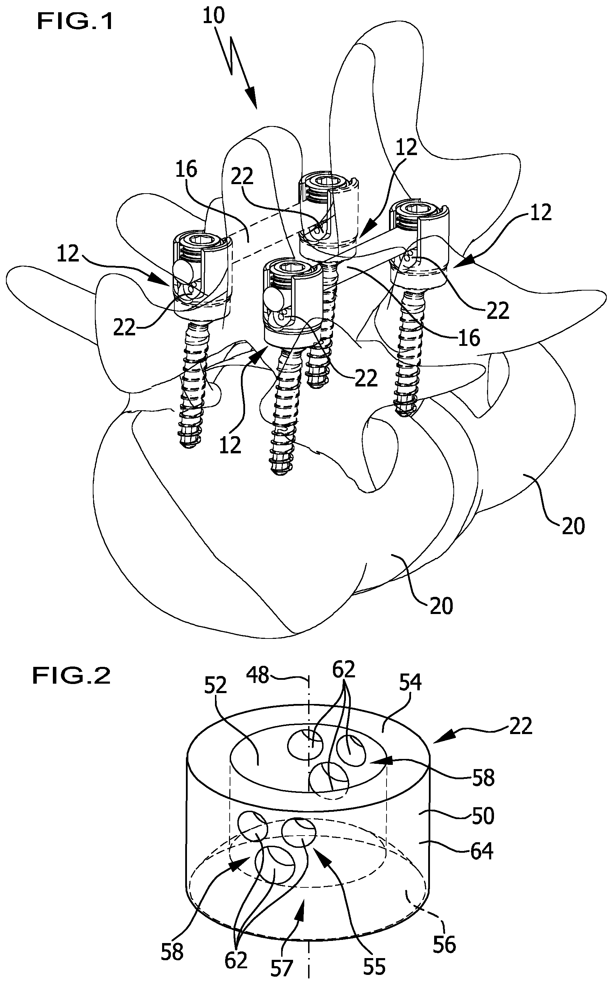

Figur 1 zeigt ein insgesamt mit dem Bezugszeichen 10 belegtes Fixationssystem in bevorzugter Ausführungsform der vorliegenden Erfindung. Beispielhaft sind beim Fixationssystem 10 Verankerungselemente 12 vorgesehen in Gestalt von Knochenschrauben 14.

Das Fixationssystem 10 umfasst vier Stück hiervon, wobei erfindungsgemäß mindestens eine Knochenschraube 14 vorgesehen ist.

Weiter umfasst das Fixationssystem 10 mindestens ein Stabilisierungselement 16, wobei vorliegend zwei Stabilisierungselemente 16, ausgestaltet als Stabelemente 18, vorgesehen sind. Je zwei Knochenschrauben 14 sind über ein Stabelement 18 miteinander verbunden.

Das Fixationssystem 10 dient zur Stabilisierung von Knochen, vorliegend von benachbarten Wirbelkörpern 20. Zu diesem Zweck sind die Knochenschrauben 14 insbesondere Pedikelschrauben.

Die vier Knochenschrauben 14 und die beiden Stabelemente 18 sind jeweils identisch ausgestaltet. Es wird nachfolgend nur auf eine der Knochenschrauben 14 und ein Stabelement 18 eingegangen.

Das Fixationssystem 10 umfasst ferner mindestens ein Anlageelement 22. Eine bevorzugte Ausführungsform des Anlageelementes 22 ist in den Figuren 2 bis 4 dargestellt. Günstigerweise ist jeder Knochenschraube 14 ein Anlageelement 22 zugeordnet.

Vorgesehen sein kann insbesondere, dass die Anlageelemente 22 identisch ausgestaltet sind.

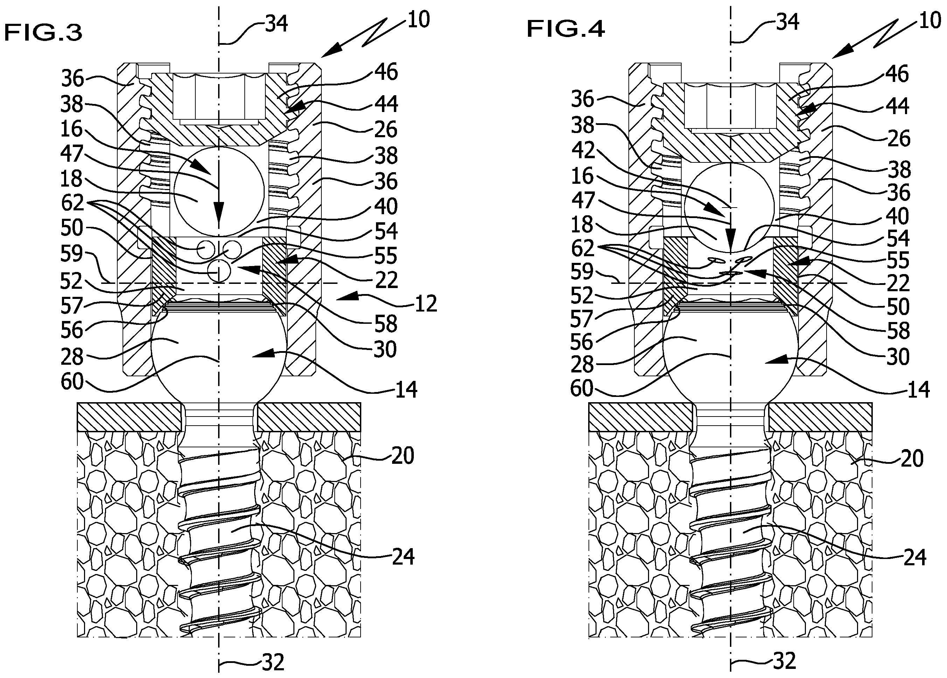

Wie insbesondere aus den Figuren 3 und 4 hervorgeht, umfasst die Knochenschraube 14 einen Verankerungsabschnitt 24 zum Verankern im Knochen und einen Aufnahmeabschnitt 26 für das Stabelement 18. Die Knochenschraube 14 ist vorliegend eine Polyaxialschraube, bei der der Aufnahmeabschnitt 26 relativ zum Verankerungsabschnitt 24 schwenkbar ist. Zu diesem Zweck umfasst der Verankerungsabschnitt 24 einen im vorliegenden Fall kugelförmigen Kopf 28. Der Kopf 28 definiert einen zumindest abschnittsweise kugelschalenförmigen Anlagebereich 30.

Der Verankerungsabschnitt 24 definiert eine Achse 32. Der Aufnahmeabschnitt 26 definiert eine Achse 34. Bei der in der Zeichnung dargestellten Relativorientierung des Verankerungsabschnittes 24 und des Aufnahmeabschnittes 26 fallen die Achsen 32, 34 zusammen.

Der Aufnahmeabschnitt 26 weist zwei im Abstand zueinander angeordnete Segmente 36 auf. An einem jeweiligen Segment 36 ist ein Innengewinde 38 angeordnet.

Zwischen den Segmenten 36 ist eine Durchgangsöffnung 40 gebildet, durch die hindurch das Stabelement 18 hindurchgeführt werden kann. Vorzugsweise stimmt eine Erstreckungsrichtung des Stabelementes 18 mit einer Achse 42 der Durchgangsöffnung 40 überein.

Zum Fixieren des Stabelementes 18 im Aufnahmeabschnitt 26 umfasst das Fixationssystem 10 jeweils ein Fixierelement 44. Das Fixierelement 44 ist vorliegend ein Schraubelement 46. Das Schraubelement 46 kann mit den Gewinden 38 der Segmente 36 verschraubt werden. Dies erlaubt es, das Stabelement 18 in einer Fixierrichtung 47 mit einer auf den Verankerungsabschnitt 24 gerichteten Fixierkraft zu beaufschlagen.

Das Anlageelement 22 ist im vorliegenden Fall zum Abstützen des Stabelementes 18 vorgesehen relativ zum Verankerungsabschnitt 24. Dabei sind das Anlageelement 22 und das Schraubelement 46 auf einander gegenüberliegenden Seiten des Stabelementes 18 angeordnet.

Das Anlageelement 22 ist getrennt vom Aufnahmeabschnitt 26 gebildet und im Aufnahmeabschnitt 26 angeordnet. Vorliegend ist das Anlageelement 22 so bemessen, dass es formschlüssig zwischen den Segmenten 36 in der Durchgangsöffnung 40 positioniert ist. Eine Außenkontur des Anlageelementes 22 ist dabei vorzugsweise an eine Innenkontur der Segmente 36 angepasst. Vorliegend sind diese jeweiligen Konturen kreisförmig bzw. kreisbogenförmig. Dies erlaubt es, das Anlageelement 22 in einer Ebene quer und insbesondere senkrecht zur Achse 34 unbeweglich im Aufnahmeabschnitt 26 zu positionieren.

Wie aus den Figuren 2 bis 4 weiter hervorgeht, ist das Anlageelement 22 im vorliegenden Beispiel hülsenförmig mit einer zentralen Durchgangsöffnung ausgestaltet und definiert dabei eine Achse 48. Im Aufnahmeabschnitt 26 ist das Anlageelement 22 koaxial zu diesem angeordnet und ausgerichtet, wobei die Achsen 34 und 48 miteinander fluchten. Je nach Relativorientierung des Verankerungsabschnittes und des Aufnahmeabschnittes 26 kann das Anlageelement 22 auch koaxial zum Verankerungsabschnitt 24 ausgerichtet sein.

Das Anlageelement 22 umfasst vorliegend eine äußere Umfangsfläche 50, eine innere Umfangsfläche 52, an einer dem Stabelement 18 zugewandten Stirnseite einen Anlagebereich 54 und an einer dem Kopf 28 zugewandten Stirnseite einen Anlagebereich 56.

Das Anlageelement 22 umfasst einen ersten Anlageelementabschnitt 55 und einen zweiten Anlageelementabschnitt 57. Der erste Anlageelementabschnitt 55 ist dem Stabelement 18 zugewandt und bildet stirnseitig den Anlagebereich 54. Der zweite Anlageelementabschnitt 57 ist dem Verankerungsabschnitt 24, insbesondere dessen Kopf 28, zugewandt. Stirnseitig bildet der zweite Anlageelementabschnitt 57 den Anlagebereich 56.

Bezogen auf die Fixierrichtung 47 ist der erste Anlageelementabschnitt 55 proximal bezüglich des Stabelementes 18 angeordnet und der zweite Anlageelementabschnitt distal bezüglich des Stabelementes 18. In Bezug auf den Verankerungsabschnitt 24, insbesondere den Kopf 28, gilt das Entgegengesetzte.

Zum Zwecke der Erläuterung ist in den Figuren 3 und 4 eine fiktive Trennebene zwischen den Anlageelementabschnitten mittels einer gestrichelten Linie 59 eingezeichnet. Die Anlageelementabschnitte 55 und 57 gehen indessen vorliegend, insbesondere hinsichtlich ihrer Verformbarkeit, längs der Fixierrichtung 47 ineinander über. Ein diesbezüglicher Übergangsabschnitt ist in der Zeichnung nicht gesondert dargestellt.

Bei einer andersartigen vorteilhaften Ausführungsform könnte vorgesehen sein, dass zwischen den Anlageelementabschnitten 55 und 57, insbesondere hinsichtlich ihrer Verformbarkeit, eine diskrete Abschnittsgrenze vorgesehen ist. Die Abschnittsgrenze ist beispielsweise quer und insbesondere senkrecht zur Fixierrichtung 47 ausgerichtet (vorliegend quer und insbesondere senkrecht zur Zeichenebene).

Der Anlagebereich 56 ist kugelschalenförmig ausgestaltet und in seiner Form an den Anlagebereich 30 angepasst. Bei einer Verschwenkung des Aufnahmeabschnitts 26 relativ zum Verankerungsabschnitt 24, solange das Fixierelement 44 unfixiert ist, wird dadurch auch das Anlageelement 22 mit verschwenkt, wobei der Anlagebereich 56 stets in flächigem Kontakt mit dem Kopf 28 bleibt.

Der Anlagebereich 54 ist vorliegend planar ausgestaltet und durch die ringförmige Stirnfläche des Anlageelementes 22 gebildet. Das Stabelement 18 kann an dem Anlagebereich 54 anliegen und insbesondere darauf aufliegen (Figuren 3 und 4).

Das Anlageelement 22 umfasst mindestens einen Verformungsbereich 58. Vorliegend sind zwei Verformungsbereiche 58 vorgesehen, die an einander bezüglich der Achse 48 diametral gegenüberliegenden Abschnitten des Anlageelementes 22 angeordnet sind.

Beim Anlageelement 22 umfasst der erste Anlageelementabschnitt 55 den Verformungsbereich 58 oder bildet den mindestens einen Verformungsbereich 58 aus. Demgegenüber umfasst oder bildet der zweite Anlageelementabschnitt 57 vorliegend keinen Verformungsbereich.

Infolge des mindestens einen Verformungsbereiches 58 (vorliegend zwei Verformungsbereiche) weist der erste Anlageelementabschnitt 55 eine höhere Verformbarkeit auf als der zweite Anlageelementabschnitt 57. Die Verformung erfolgt aufgrund der Fixierkraft des Fixierelementes 44 und ermöglicht eine Anpassung des Anlageelementes 22 an das Stabelement 18. Dies wird nachfolgend erläutert.

Demgegenüber ist der zweite Anlageelementabschnitt 57 "härter" oder "steifer" als der erste Anlageelementabschnitt 55. Dies ermöglicht einen zuverlässigen Sitz an dem Kopf 28, wodurch beispielsweise der vorstehend erwähnte flächige Kontakt über den Anlagebereich 56 sichergestellt werden kann.

Der zweite Anlageelementabschnitt 57 kann bei den mit dem Fixierelement 44 üblicherweise auftretenden Fixierkräften wie vorstehend erläutert insbesondere unverformt oder im Wesentlichen unverformt sein.

Die Verformbarkeit kann vom ersten Anlageelementabschnitt 55 zum zweiten Anlageelementabschnitt 57 über den vorstehend genannten Übergangsabschnitt beispielsweise graduell abnehmen. Ist wie vorstehend erwähnt eine Abschnittsgrenze vorgesehen, kann beispielsweise eine stufenweise Veränderung der Verformbarkeit vom ersten zum zweiten Anlageelementabschnitt 55, 57 auftreten.

Die Verformungsbereiche 58 sind symmetrisch bezüglich einer ersten, die Achsen 34, 48 enthaltenden Ebene relativ zueinander ausgestaltet. Diese Ebene verläuft in den Figuren 3 und 4 in der Zeichenebene. Darüber hinaus sind die Verformungsbereiche 58 in sich symmetrisch bezüglich einer die Achsen 34, 48 enthaltenden Ebene ausgebildet. Hierbei handelt es sich um eine Ebene 60 senkrecht zur Zeichenebene in den Figuren 3 und 4, wobei die Symmetrieebene 60 eine Mittelebene des Aufnahmeabschnittes 26 im bestimmungsgemäßen Gebrauch des Fixationssystems 10 ist.

Die Verformungsbereiche 58 sind unterhalb des Anlagebereiches 54 angeordnet.

An einem jeweiligen Verformungsbereich 58 ist mindestens eine Materialausnehmung vorhanden. Vorliegend umfasst jeder Verformungsbereich 58 drei Materialausnehmungen, ausgestaltet als Durchgangsöffnungen 62 des Anlageelementes 22. Die Durchgangsöffnungen 62 weisen einen runden und insbesondere kreisrunden Querschnitt auf.

Hiervon sind zwei Durchgangsöffnungen 62 identisch ausgestaltet und symmetrisch bezüglich der Ebene 60 zueinander angeordnet. Eine dritte Durchgangsöffnung 62 ist zu diesen beiden Durchgangsöffnungen 62 auf Lücke angeordnet und in sich symmetrisch bezüglich der Ebene 60. Die letztgenannte Durchgangsöffnung 62 weist einen größeren Durchmesser auf als die erstgenannten Durchgangsöffnungen 62.

Die Durchgangsöffnungen 62 an den einander bezüglich der Achse 48 gegenüberliegenden Verformungsbereichen 58 fluchten jeweils. Dabei sind die Durchgangsöffnungen 62 jeweils in der Erstreckungsrichtung des Stabelementes 18 ausgerichtet. Vorzugsweise sind die Durchgangsöffnungen 62 parallel zur Achse 42 ausgerichtet.

Die Durchgangsöffnungen 62 verlaufen vorliegend parallel zu einer vom Anlagebereich 54 definierten Ebene.

Im Gebrauch des Fixationssystems 10 wird das Stabelement 18 vom Schraubelement 46 mit einer auf das Anlageelement 22 und über dieses auf den Kopf 28 gerichteten Fixierkraft beaufschlagt (Figur 4). Die Fixierkraft führt zu einer Verformung des Anlageelementes 22 an den Verformungsbereichen 58. Vorzugsweise kann dabei eine gezielte Verformung erzielt werden.

Über die Verformung besteht die Möglichkeit, Stabelemente 18 unterschiedlicher Beschaffenheit, insbesondere unterschiedlichen Materials und/oder unterschiedlichen Durchmessers, an der Knochenschraube 14 zu adaptieren. Gesonderte Knochenschrauben 14 sind nicht erforderlich. Die Vielseitigkeit des Fixationssystems 10 wird dadurch erhöht.

Durch die Verformung des Anlageelementes 22 kann insbesondere ein flächiger Kontaktbereich zwischen dem Stabelement 18 und dem Anlageelement 22 erzielt werden. Dies begünstigt die Vergleichmäßigung der Pressung und hilft es, Punktkontakte und Linienkontakte zu vermeiden. Auf diese Weise ist eine zuverlässigere Fixierung des Stabelementes 18 sichergestellt. Etwaigen Korrosionsbildungen wird entgegengewirkt.

Über den im Verhältnis zum Anlageelementabschnitt 55 steiferen Anlageelementabschnitt 57 wird eine zuverlässige Passung in Bezug auf den Kopf 28 sichergestellt.

Die Verformung des Anlageelementes 22 kann plastisch oder elastisch sein.

Das Anlageelement 22 ist vorzugsweise einstückig ausgebildet.

In Bezug auf vorteilhafte Materialien und die bei Anwendung des Fixationssystems 10 auftretenden Kräfte wird auf die voranstehenden Ausführungen verwiesen.

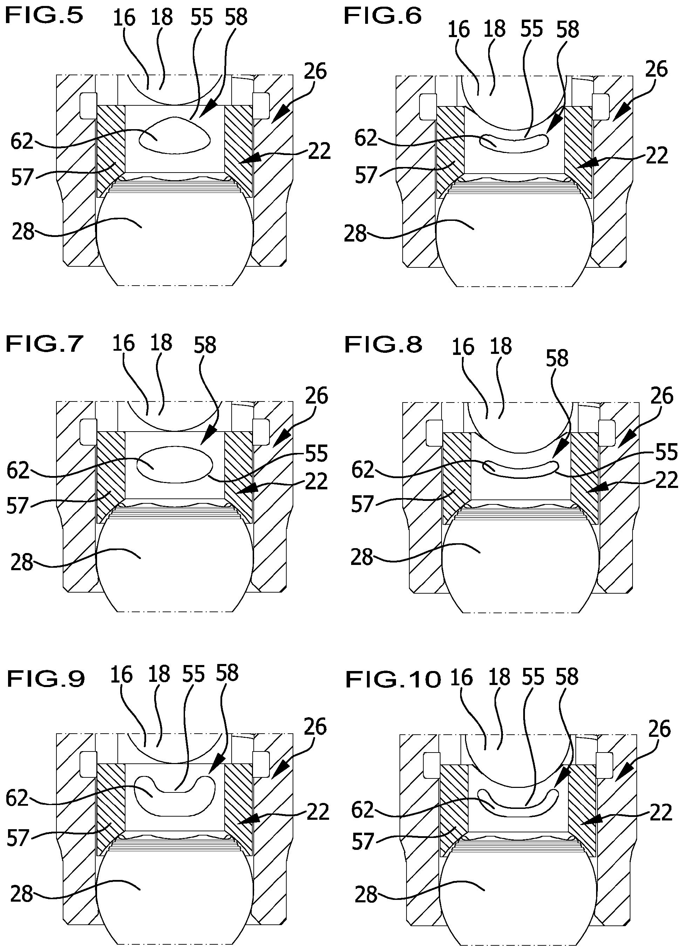

Nachfolgend wird unter Verweis auf die Figuren 5 bis 18 auf weitere bevorzugte Ausführungsformen der Erfindung eingegangen. Dabei kommt jeweils ein anstelle des in den Figuren 1 bis 4 dargestellten Anlageelementes 22 andersartiges Anlageelement zum Einsatz. Dargestellt ist der Einsatz jeweils mit der Knochenschraube 14 und dem Stabelement 18.

Die voranstehend erläuterten Vorteile können unter Einsatz der nachfolgend beschriebenen Anlageelemente ebenfalls erzielt werden, so dass diesbezüglich auf die voranstehenden Ausführungen verwiesen werden kann. Die Darstellung der Figuren 5 und 6, 7 und 8, 9 und 10 sowie 12 und 13 entspricht der Darstellung in den Figuren 3 bzw. 4 in einer Teilansicht.

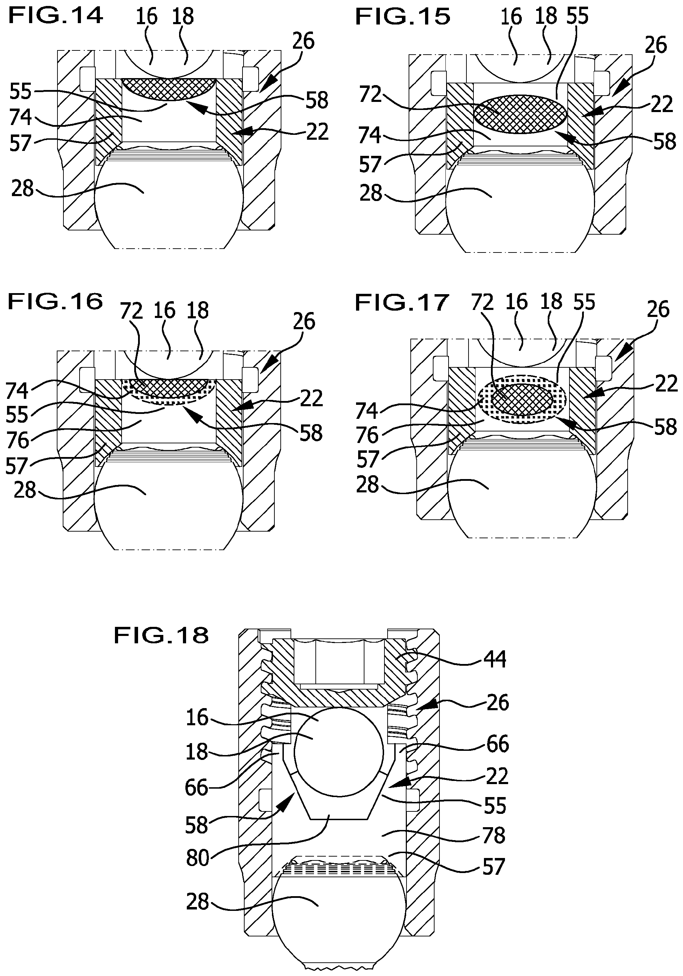

Die Darstellungen der Figuren 14 bis 18 entsprechen der Darstellung gemäß Figur 3 in einer Teilansicht. Hierbei ist das jeweilige Anlageelement 22 nicht mit der Fixierkraft beaufschlagt, sondern für eine deutlichere Darstellung im unbeaufschlagten Zustand gezeigt.

Die in den Figuren 5 bis 18gezeigten Ausführungsformen weisen jeweils ein Anlageelement 22 auf, das vorzugsweise die Anlageelementabschnitte 55 und 57 für das Stabelement 18 bzw. für den Verankerungsabschnitt 24, insbesondere den Kopf 28, aufweist. Hierbei ist jeweils die Verformbarkeit am Anlageelementabschnitt 55 höher als am Anlageelementabschnitt 57, wobei der Anlageelementabschnitt 55 jeweils mindestens einen Verformungsbereich 58 umfasst oder ausbildet.

Das Anlageelement 22 gemäß den Figuren 5 und 6 weist nur eine DurchgangsÖffnung 62 am jeweiligen Verformungsbereich 58 auf. Die Durchgangsöffnung 62 ist im nicht beaufschlagten Zustand ungefähr von der Form eines abgerundeten, in die Breite gezogenen gleichschenkligen Dreiecks, näherungsweise einer Wankelform. Im beaufschlagten Zustand ist die Durchgangsöffnung 62 abhängig von der Fixierkraft beispielsweise bogenförmig.

Auch bei den in den Figuren 7 bis 10 dargestellten Ausführungsformen ist jeweils nur eine Durchgangsöffnung 62 des Verformungsbereiches 58 vorgesehen.

Bei der Ausführungsform gemäß den Figuren 7 und 8 ist die Durchgangsöffnung 62 im nicht beaufschlagten Zustand elliptisch. Im beaufschlagten Zustand ist die Durchgangsöffnung 62 abhängig von der Fixierkraft beispielsweise bogenförmig.

Die Durchgangsöffnung 62 bei der Ausführungsform gemäß den Figuren 9 und 10 ist im nicht beaufschlagten Zustand ungefähr C-förmig, jeweils mit in Richtung auf das Stabelement 18 weisenden Enden des "C". Im beaufschlagten Zustand weist die Durchgangsöffnung 62 ungefähr die Form eines U auf mit in Richtung auf das Stabelement 18 weisenden Schenkeln.

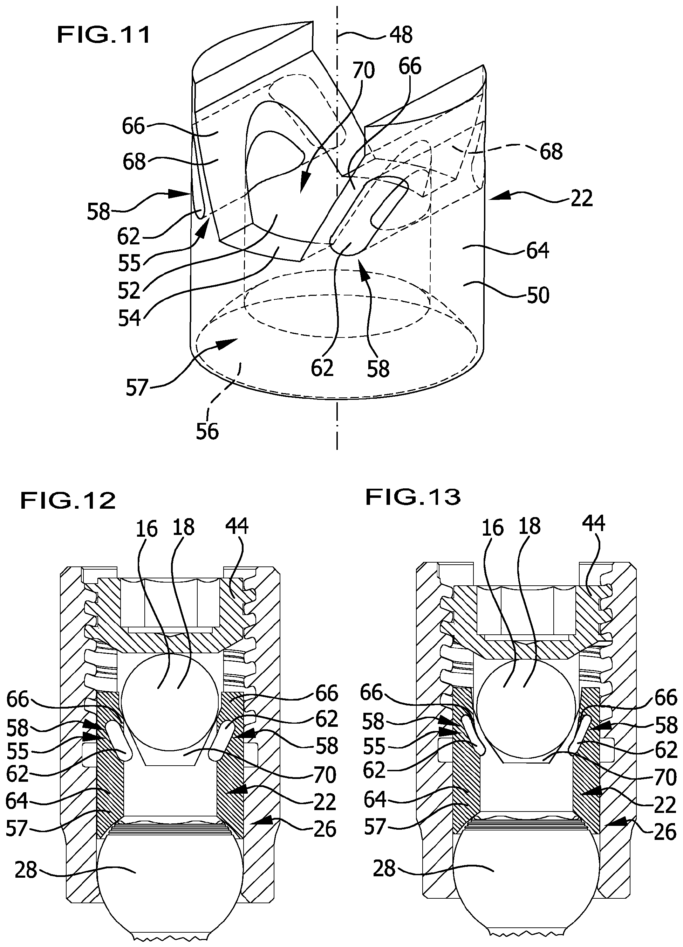

Figur 11 zeigt eine Ausführungsform des Anlageelementes 22, die einen hülsenförmigen Abschnitt 64 umfasst. Vom Abschnitt 64 stehen einander bezüglich der Achse 48 gegenüberliegend zwei Stützglieder 66 ab. Ein jeweiliges Stützglied 66 bildet einen seitlichen Anlagebereich 68 für das Stabelement 18.

An einem jeweiligen Stützglied 66 ist ein Verformungsbereich 58 angeordnet. Der Verformungsbereich 58 weist jeweils eine Durchgangsöffnung 62 mit langlochförmigem Querschnitt auf, die parallel zur Achse 42 verläuft.

Zwischen den Stützgliedern 66 ist eine Vertiefung 70 angeordnet, die sich in Einführrichtung des Stabelementes 18 verjüngt. Über die Vertiefung 70 ist sichergestellt, dass das Stabelement 18 relativ zum Aufnahmeabschnitt 26 zentriert ausgerichtet ist.

Das Stabelement 18 kann die Anlagebereiche 68 kontaktieren. Vorgesehen sein kann auch ein Kontakt des Stabelementes 18 mit dem Anlagebereich 54 (nicht gezeigt).

Bei den bislang beschriebenen Ausführungsformen umfasst der Verformungsbereich 58 jeweils mindestens eine Materialausnehmung, insbesondere in Gestalt der Durchgangsöffnung 62.

Abweichend hiervon weist das jeweilige in den Figuren 14 bis 18 dargestellte Anlageelement 22 keine Materialausnehmung auf. Stattdessen wird der jeweilige Verformungsbereich 58 über Verformungsabschnitte des Anlageelementes 22 mit voneinander unterschiedlichen Verformbarkeiten gebildet.

Bei den Darstellungen gemäß den Figuren 14 bis 17 sind jeweils zwei einander bezüglich der Achse 48 gegenüberliegende Verformungsbereiche 58 vorgesehen. Hiervon zeigt die Zeichnung nur einen Verformungsbereich 58.

Das Anlageelement 22 gemäß Figur 14 umfasst einen ersten Verformungsabschnitt 72 und einen zweiten Verformungsabschnitt 74. Der zweite Verformungsabschnitt 74 wird durch den hülsenförmigen Grundkörper des Anlageelementes 22 gebildet, von dem an der dem Stabelement 18 zugewandten Seite abschnittsweise eine konkave Ausnehmung ausgespart ist. Diese Ausnehmung wird durch den ersten Verformungsabschnitt 72 ausgefüllt.

Die Verformbarkeit des ersten Verformungsabschnittes 72 ist höher als die Verformbarkeit des zweiten Verformungsabschnittes 74. Bei einer Kraftbeaufschlagung verformt sich das Anlageelement 22 am ersten Verformungsabschnitt 72 dadurch stärker als am zweiten Verformungsabschnittes 74, zur Adaption des Stabelementes 18.

Bei dem Anlageelement 22 gemäß Figur 15 sind ebenfalls zwei Verformungsabschnitten 72, 74 vorgesehen.

Während der zweite Verformungsabschnitt 74 den ersten Verformungsabschnitt 72 bei der Ausführungsform nach Figur 14 nur abschnittsweise umgibt, ist der Verformungsabschnitt 72 vom Verformungsabschnitt 74 bei der Ausführungsform gemäß Figur 15 in Umfangsrichtung vollständig umgeben. Der Verformungsabschnitt 72 kann allerdings beispielsweise von der äußeren Umfangsfläche 50 bis zur inneren Umfangsfläche 52 reichen.

Im Querschnitt weist der Verformungsabschnitt 72 eine ungefähr elliptische Form auf.

Die Ausführungsform gemäß Figur 16 unterscheidet sich von der Ausführungsform gemäß Figur 14 dadurch, dass der erste Verformungsabschnitt 72 innerhalb einer Ausnehmung des zweiten Verformungsabschnittes 74 angeordnet ist, der seinerseits innerhalb einer Ausnehmung des einen dritten Verformungsabschnittes 76 bildenden Grundkörpers des Anlageelementes 22 angeordnet ist. Der zweite Verformungsabschnitt 74 bildet gewissermaßen einen Übergangsabschnitt vom ersten Verformungsabschnitt 72 zum dritten Verformungsabschnitt 76, wobei dieser Verformungsabschnitt 76 dem zweiten Verformungsabschnitt 74 bei der Ausführungsform gemäß Figur 14 entspricht.

Insbesondere ist die Verformbarkeit am zweiten Verformungsabschnitt 74 geringer als am ersten Verformungsabschnitt 72, und die Verformbarkeit des dritten Verformungsabschnittes 76 ist geringer als am zweiten Verformungsabschnitt 74.

Die Ausführungsform gemäß Figur 17 unterscheidet sich von der Ausführungsform gemäß 15 dadurch, dass wie bei der Ausführungsform gemäß Figur 16 drei Verformungsabschnitte 72 bis 76 vorgesehen sind. Hierbei ist der Verformungsabschnitt 72 vollständig vom Verformungsabschnitt 74 umgeben, und der Verformungsabschnitt 74 ist vollständig vom Verformungsabschnitt 76 umgeben. Die Verformbarkeit des Anlageelementes 72 steigt vom ersten Verformungsabschnitt 72 über den zweiten Verformungsabschnitten 74 bis zum dritten Verformungsabschnitt 76 an.

Vorgesehen sein kann, dass die Verformbarkeit zwischen einander benachbarten Verformungsabschnitten diskret anwächst. Eine kontinuierliche Steigerung der Verformbarkeit kann vorgesehen sein.

Das Anlageelement 22 gemäß der Ausführungsform nach Figur 18 weist einen Grundkörper 78 auf, dessen Form weitgehend mit der Form des Anlageelementes gemäß den Figuren 11 bis 13 übereinstimmt. Es ist der hülsenförmige Abschnitt 64 mit den Stützgliedern 66 vorgesehen. An den Stützgliedern 66 ist jedoch kein Verformungsbereich 58 und insbesondere keine Durchgangsöffnung 62 angeordnet.

In der Vertiefung 70 ist ein weiterer Abschnitt 80 des Anlageelementes 22 positioniert, an dem das Stabelement 18 anliegt. Der Abschnitt 80 weist eine höhere Verformbarkeit auf als der Grundkörper 78 und bildet eine wannenförmige Aufnahme für das Stabelement 18.

Bei den Ausführungsformen gemäß den Figuren 14 und 18 kann vorgesehen sein, dass die jeweiligen Abschnitte des Anlageelementes 22 separat voneinander gebildet und miteinander verbunden sind. Denkbar ist alternativ eine einstückige Ausgestaltung des jeweiligen Anlageelementes 22.

### Bezugszeichenliste:

- 10: Fixationssystem
- 12: Verankerungselement
- 14: Knochenschraube
- 16: Stabilisierungselement
- 18: Stabelement
- 20: Wirbelkörper
- 22: Anlageelement
- 24: Verankerungsabschnitt
- 26: Aufnahmeabschnitt
- 28: Kopf
- 30: Anlagebereich
- 32, 34: Achse
- 36: Segment
- 38: Innengewinde
- 40: Durchgangsöffnung
- 42: Achse
- 44: Fixierelement
- 47: Fixierrichtung
- 46: Schraubelement
- 48: Achse
- 50, 52: Umfangsfläche
- 54, 56: Anlagebereich
- 55, 57: Anlageelementabschnitt
- 58: Verformungsbereich
- 59: Trennebene
- 60: Symmetrieebene
- 62: Durchgangsöffnung
- 64: Abschnitt
- 66: Stützglied
- 68: Anlagebereich
- 70: Vertiefung
- 72, 74, 76: Verformungsabschnitt
- 78: Grundkörper
- 80: Abschnitt

## Patentansprüche

1. Chirurgisches Fixationssystem, umfassend mindestens ein Verankerungselement (12) mit einem Verankerungsabschnitt (24) zum Verankern an einem Knochen (20) und mit einem Aufnahmeabschnitt (26) für ein Stabilisierungselement (16) zur Verbindung mit einem weiteren Verankerungselement (12), wobei das Stabilisierungselement (16) im Aufnahmeabschnitt (26) anordenbar und darin mittels eines Fixierelementes (44) fixierbar ist, wobei das Fixationssystem (10) ein am Aufnahmeabschnitt (26) angeordnetes und am Verankerungsabschnitt (24) anliegendes Anlageelement (22) zum Anlegen des Stabilisierungselementes (16) umfasst, wobei das Anlageelement (22) mindestens einen Verformungsbereich (58) zum Verformen abhängig von einer das Stabilisierungselement (16) beaufschlagenden Fixierkraft des Fixierelementes (44) aufweist, wobei das Anlageelement (22) zumindest abschnittsweise hülsenförmig ausgestaltet ist und an einer Stirnseite einen Anlagebereich (54) für das Stabilisierungselement (16) aufweist, wobei mindestens ein Verformungsbereich (58) unterhalb des Anlagebereiches (54) des Anlageelementes (22) für das Stabilisierungselement (16) angeordnet ist, **dadurch gekennzeichnet, dass** der mindestens eine Verformungsbereich (58) durch mindestens eine Materialausnehmung am Anlageelement (22) gebildet ist oder eine solche umfasst.

2. Fixationssystem nach Anspruch 1, **dadurch gekennzeichnet, dass** das Anlageelement (22)
a) getrennt vom Aufnahmeabschnitt (26) gebildet und in dem Aufnahmeabschnitt (26) angeordnet ist oder dass der Aufnahmeabschnitt (26) das Anlageelement (22) umfasst oder ausbildet und/oder
b) am mindestens einen Verformungsbereich (58) elastisch oder plastisch verformbar ausgebildet ist.

3. Fixationssystem nach einem der voranstehenden Ansprüche, **dadurch gekennzeichnet, dass** mindestens ein Verformungsbereich (58) an einem Anlagebereich (54) des Anlageelementes (22) für das Stabilisierungselement (16) angeordnet ist.

4. Fixationssystem nach einem der voranstehenden Ansprüche, **dadurch gekennzeichnet, dass** mindestens ein Verformungsbereich (58) an oder seitlich neben einem seitlichen Anlagebereich (68) für das Stabilisierungselement (16) angeordnet ist.

5. Fixationssystem nach einem der voranstehenden Ansprüche, **dadurch gekennzeichnet, dass** das Anlageelement (22) einen dem Stabilisierungselement (16) zugewandten ersten Anlageelementabschnitt (55) und einen dem Verankerungsabschnitt (24) zugewandten zweiten Anlageelementabschnitt (57) umfasst, wobei der erste Anlageelementabschnitt (55) den mindestens einen Verformungsbereich (58) umfasst oder ausbildet und eine höhere Verformbarkeit infolge der Fixierkraft aufweist als der zweite Anlageelementabschnitt (57).

6. Fixationssystem nach Anspruch 5, **gekennzeichnet durch**
a) eine hinsichtlich der Verformbarkeit des Anlageelementes (22) diskrete Abschnittsgrenze zwischen dem ersten Anlageelementabschnitt (55) und dem zweiten Anlageelementabschnitt (57), wobei insbesondere die Abschnittsgrenze quer und insbesondere senkrecht zu einer Fixierrichtung des Fixierelementes (44) in Richtung des Verankerungsabschnittes (24) ausgerichtet ist,
oder
b) einen hinsichtlich der Verformbarkeit des Anlageelementes (22) vorhandenen Übergangsabschnitt, über den der erste Anlageelementabschnitt (55) und der zweite Anlageelementabschnitt (57) ineinander übergehen.

7. Fixationssystem nach einem der voranstehenden Ansprüche, **dadurch gekennzeichnet, dass** der mindestens eine Verformungsbereich (58) eine Erstreckung parallel zu einem Anlagebereich (54) des Anlageelementes (22) für das Stabilisierungselement (16) aufweist.

8. Fixationssystem nach einem der voranstehenden Ansprüche, **dadurch gekennzeichnet, dass** der mindestens eine Verformungsbereich (58) symmetrisch am Anlageelement (22) angeordnet oder gebildet ist bezüglich einer eine Achse (34) des Aufnahmeabschnittes und/oder des Anlageelementes (22) enthaltenden Symmetrieebene (60).

9. Fixationssystem nach Anspruch 8, **dadurch gekennzeichnet, dass** die Materialausnehmung
a) eine Ausnehmung an einer Oberfläche des Anlageelementes (22) ist, wobei das Anlageelement (22) am Rand der Ausnehmung verformbar ist,
oder
b) eine Durchgangsöffnung (62) des Anlageelementes (22) ist oder umfasst,
wobei insbesondere die Durchgangsöffnung (62) einen kreisrunden, elliptischen, ovalen, runden, langlochförmigen oder einen unrunden Querschnitt aufweist,
oder
c) ein im Anlageelement (22) gebildeter, allseits umschlossener Hohlraum ist,
wobei insbesondere der Hohlraum einen kreisrunden, elliptischen, ovalen, runden, langlochförmigen oder einen unrunden Querschnitt aufweist.

10. Fixationssystem nach Anspruch 8 oder 9, **dadurch gekennzeichnet, dass** die Materialausnehmung
a) eine Erstreckung entlang des im Aufnahmeabschnitt (26) angeordneten Stabilisierungselementes (16) aufweist
und/oder
b) eine Erstreckung radial zu einer vom Anlageelement (22) definierten Achse (48) aufweist.

11. Fixationssystem nach einem der voranstehenden Ansprüche, **dadurch gekennzeichnet, dass** das Anlageelement (22) zwei oder mehr Verformungsabschnitte (72, 74, 76) umfasst, die hinsichtlich ihrer Verformbarkeit unterschiedliche Materialen umfassen oder aus unterschiedlichen Materialien gefertigt sind, wobei der mindestens eine Verformungsbereich (58) aufgrund der materialmäßig unterschiedlichen Beschaffenheit der zwei oder mehr Verformungsabschnitte (72, 74, 76) gebildet ist, wobei insbesondere
a) die zwei oder mehr Verformungsabschnitte (72, 74, 76) getrennt voneinander gebildete und aneinander gefügte Verformungsabschnitte des Anlageelementes (22) sind,
wobei insbesondere ein erster Verformungsabschnitt (72) vorgesehen ist, der zumindest teilweise von mindestens einem zweiten Verformungsabschnitt (74) umgeben ist, wobei die Verformbarkeit des ersten Verformungsabschnittes (72) größer ist als die Verformbarkeit des mindestens einen zweiten Verformungsabschnittes (74),
und/oder
b) drei oder mehr Verformungsabschnitte (72, 74, 76) vorgesehen sind, wobei die Verformbarkeit eines jeweiligen Verformungsabschnittes (72, 74, 76), der einen weiteren Verformungsabschnitt (72, 74, 76) zumindest teilweise umgibt, geringer ist als die Verformbarkeit des umgebenen Verformungsabschnittes (72, 74, 76).

12. Fixationssystem nach einem der voranstehenden Ansprüche, **dadurch gekennzeichnet, dass** das Anlageelement (22) eine Mehrzahl von Verformungsbereichen (58) umfasst, insbesondere mit einer Mehrzahl von Materialausnehmungen,
wobei insbesondere
a) zwei oder mehr identisch ausgestaltete Verformungsbereiche (58) vorgesehen sind und/oder wobei zwei oder mehr unterschiedlich ausgestaltete Verformungsbereiche (58) vorgesehen sind und/oder
b) zwei Verformungsbereiche (58) vorgesehen sind, die im Abstand zueinander und miteinander fluchtend am Anlageelement (22) angeordnet sind.

13. Fixationssystem nach einem der voranstehenden Ansprüche, **dadurch gekennzeichnet, dass** das Anlageelement (22)
a) zwei einander diametral gegenüberliegende Verformungsbereiche (58) aufweist
und/oder
b) zwei im Abstand zueinander angeordnete Stützglieder (66) umfasst, die eine sich verjüngende Vertiefung (70) seitlich begrenzen, wobei das Stabilisierungselement (16) zwischen den Stützgliedern (66) in der Vertiefung (70) positionierbar ist, wobei vorzugsweise an zumindest einem Stützglied (66) ein Verformungsbereich (58) angeordnet ist,
und/oder
c) koaxial zum Aufnahmeabschnitt (26) und/oder zum Verankerungsabschnitt (24) ausgerichtet oder ausrichtbar ist.

14. Fixationssystem nach einem der voranstehenden Ansprüche, **dadurch gekennzeichnet, dass** der Verankerungsabschnitt (24) und das Anlageelement (22) aneinander angepasste, insbesondere zumindest abschnittsweise kugelschalenförmige Anlagebereiche (30, 56) umfassen.

15. Fixationssystem nach einem der voranstehenden Ansprüche, **dadurch gekennzeichnet, dass**
a) das Fixationssystem mindestens eines der Folgenden umfasst:
- zwei oder mehr Verankerungselemente (12);
- zwei oder mehr Anlageelemente (22);
- mindestens ein Stabilisierungselement (16), insbesondere eine Mehrzahl von Stabilisierungselementen (16);
- mindestens ein Fixierelement (44), insbesondere eine Mehrzahl von Fixierelementen (44);
und/oder
b) zumindest eines der Folgenden gilt:
- das mindestens eine Verankerungselement (12) ist eine Knochenschraube (14);
- das mindestens eine Stabilisierungselement (16) ist ein Stabelement (18);
- das mindestens eine Fixierelement (44) ist ein mit dem Aufnahmeabschnitt (26) verschraubbares Schraubelement (46).

## Claims

1. Surgical fixation system, comprising at least one anchoring element (12) with an anchoring portion (24) for anchoring to a bone (20) and with a receiving portion (26) for a stabilization element (16) for connecting to a further anchoring element (12), wherein the stabilization element (16) is arrangeable in the receiving portion (26) and is fixable therein by means of a fixing element (44), wherein the fixation system (10) comprises an abutment element (22) that is arranged on the receiving portion (26) and abuts against the anchoring portion (24) for the placement of the stabilization element (16), wherein the abutment element (22) has at least one deformation region (58) for deforming in dependence on a fixing force of the fixing element (44) acting on the stabilization element (16), wherein the abutment element (22) is of sleeve-shaped configuration at least in sections and has on an end face an abutment region (54) for the stabilization element (16), wherein at least one deformation region (58) is arranged beneath the abutment region (54) of the abutment element (22) for the stabilization element (16), **characterized in that** the at least one deformation region (58) is formed by or comprises at least one material recess on the abutment element (22).

2. Fixation system in accordance with Claim 1, **characterized in that** the abutment element (22)
a) is formed separate from the receiving portion (26) and is arranged in the receiving portion (26) or **in that** the receiving portion (26) comprises or forms the abutment element (22) and/or
b) is of elastically or plastically deformable configuration at the at least one deformation region (58).

3. Fixation system in accordance with any one of the preceding Claims, **characterized in that** at least one deformation region (58) is arranged at an abutment region (54) of the abutment element (22) for the stabilization element (16).

4. Fixation system in accordance with any one of the preceding Claims, **characterized in that** at least one deformation region (58) is arranged at or laterally next to a lateral abutment region (68) for the stabilization element (16).

5. Fixation system in accordance with any one of the preceding Claims, **characterized in that** the abutment element (22) comprises a first abutment element portion (55) facing toward the stabilization element (16) and a second abutment portion (57) facing toward the anchoring portion (12), wherein the first abutment element portion (55) comprises or forms the at least one deformation region (58) and has a higher deformability as a result of the fixing force than the second abutment element portion (57).

6. Fixation system in accordance with Claim 5, **characterized by**
a) a portion boundary, which is discrete with respect to the deformability of the abutment element (22), between the first abutment element portion (55) and the second abutment element portion (57),
wherein, in particular, the portion boundary is oriented transversely and, in particular, perpendicularly to a fixing direction of the fixing element (44) in the direction of the anchoring portion (24),
or
b) a transition portion, which is present with respect to the deformability of the abutment element (22), by way of which the first abutment element portion (55) and the second abutment element portion (57) merge into one another.

7. Fixation system in accordance with any one of the preceding Claims, **characterized in that** the at least one deformation region (58) has an extent in parallel to an abutment region (54) of the abutment element (22) for the stabilization element (16).

8. Fixation system in accordance with any one of the preceding Claims, **characterized in that** the at least one deformation region (58) is arranged or formed symmetrically on the abutment element (22) with respect to a symmetry plane (60) containing an axis (34) of the receiving portion and/or the abutment element (22).

9. Fixation system in accordance with Claim 8, **characterized in that** the material recess
a) is a recess on a surface of the abutment element (22), wherein the abutment element (22) is deformable at the rim of the recess. or
b) is or comprises a through-opening (62) of the abutment element (22),
wherein, in particular, the through-opening (62) has a circular, elliptical, oval, round, elongate hole-shaped, or non-round cross section,
or
c) is a cavity that is formed in the abutment element (22) and is enclosed on all sides,
wherein, in particular, the cavity has a circular, elliptical, oval, round, elongate hole-shaped, or non-round cross section.

10. Fixation system in accordance with Claim 8 or 9, **characterized in that** the material recess
a) has an extent along the stabilization element (16) arranged in the receiving portion (26)
and/or
b) has an extent radially to an axis (48) defined by the abutment element (22).

11. Fixation system in accordance with any one of the preceding Claims, **characterized in that** the abutment element (22) comprises two or more deformation portions (72, 74, 76), which comprise or are made of different materials with respect to their deformability, wherein the at least one deformation region (58) is formed as a result of the materially different quality of the two or more deformation portions (72, 74, 76), wherein, in particular,
a) the two or more deformation portions (72, 74, 76) are deformation portions of the abutment element (22) that are formed separate from one another and are joined to one another,
wherein, in particular, a first deformation portion (72) is provided, which is at least partially surrounded by at least one second deformation portion (74), wherein the deformability of the first deformation portion (72) is greater than the deformability of the at least one second deformation portion (74),
and/or
b) three or more deformation portions (72, 74, 76) are provided, wherein the deformability of a respective deformation portion (72, 74, 76) that at least partially surrounds a further deformation portion (72, 74, 76) is lesser than the deformability of the surrounded deformation portion (72, 74, 76).

12. Fixation system in accordance with any one of the preceding Claims, **characterized in that** the abutment element (22) comprises a plurality of deformation regions (58), in particular with a plurality of material recesses,
wherein, in particular,
a) two or more identically configured deformation regions (58) are provided and/or wherein two or more differently configured deformation regions (58) are provided
and/or
b) two deformation regions (58) are provided, which are arranged on the abutment element (22) at a distance from one another and in alignment with one another.

13. Fixation system in accordance with any one of the preceding Claims, **characterized in that** the abutment element (22)
a) has two diametrically opposed deformation regions (58) and/or
b) comprises two support members (66) arranged at a distance from one another, which laterally delimit a tapering depression (70), wherein the stabilization element (16) is positionable between the support members (66) in the depression (70), wherein a deformation region (58) is preferably arranged on at least one support member (66),
and/or
c) is oriented or orientable coaxially to the receiving portion (26) and/or to the anchoring portion (24).

14. Fixation system in accordance with any one of the preceding Claims, **characterized in that** the anchoring portion (24) and the abutment element (22) comprise abutment regions (30, 56) that are adapted to one another and, in particular, are spherical cup-shaped at least in sections.

15. Fixation system in accordance with any one of the preceding Claims, **characterized in that**
a) the fixation system comprises at least one of the following:
- two or more anchoring elements (12);
- two or more abutment elements (22);
- at least one stabilization element (16), in particular a plurality of stabilization elements (16);
- at least one fixing element (44), in particular a plurality of fixing elements (44);
and/or
b) at least one of the following applies:
- the at least one anchoring element (12) is a bone screw (14);
- the at least one stabilization element (16) is a rod element (18);
- the at least one fixing element (44) is a screw element (46) that is screwable to the receiving portion (26).

## Revendications

1. Système de fixation chirurgical comprenant au moins un élément d'ancrage (12) avec une partie d'ancrage (24) pour l'ancrage à un os (20) et avec une partie de réception (26) pour un élément de stabilisation (16) destiné à être relié à un autre élément d'ancrage (12), l'élément de stabilisation (16) pouvant être agencé dans la partie de réception (26) et y être fixé au moyen d'un élément de fixation (44), le système de fixation (10) comprenant un élément d'appui (22) agencé sur la partie de réception (26) et s'appuyant contre la partie d'ancrage (24) pour appliquer l'élément de stabilisation (16), l'élément d'appui (22) présentant au moins une zone de déformation (58) pour se déformer en fonction d'une force de fixation de l'élément de fixation (44) agissant sur l'élément de stabilisation (16), l'élément d'appui (22) étant conçu au moins par parties en forme de manchon et présentant sur un côté frontal une zone d'appui (54) pour l'élément de stabilisation (16), au moins une zone de déformation (58) étant agencée en dessous de la zone d'appui (54) de l'élément d'appui (22) pour l'élément de stabilisation (16), **caractérisé en ce que** l'au moins une zone de déformation (58) est formée par au moins un évidement de matière sur l'élément d'appui (22) ou comprend un tel évidement.

2. Système de fixation selon la revendication 1, **caractérisé en ce que** l'élément d'appui (22)
a) est formé séparément de la partie de réception (26) et est agencé dans la partie de réception (26) ou **en ce que** la partie de réception (26) comprend ou forme l'élément d'appui (22)
et/ou
b) est conçu de manière à être déformable de manière élastique ou plastique au niveau d'au moins une zone de déformation (58).

3. Système de fixation selon l'une des revendications précédentes, **caractérisé en ce qu'**au moins une zone de déformation (58) est agencée au niveau d'une zone d'appui (54) de l'élément d'appui (22) pour l'élément de stabilisation (16).

4. Système de fixation selon l'une des revendications précédentes, **caractérisé en ce qu'**au moins une zone de déformation (58) est agencée sur ou latéralement à côté d'une zone d'appui latérale (68) pour l'élément de stabilisation (16).

5. Système de fixation selon l'une des revendications précédentes, **caractérisé en ce que** l'élément d'appui (22) comprend une première partie d'élément d'appui (55) tournée vers l'élément de stabilisation (16) et une deuxième partie d'élément d'appui (57) tournée vers la partie d'ancrage (24), la première partie d'élément d'appui (55) comprenant ou formant au moins une zone de déformation (58) et présentant une déformabilité plus élevée que la deuxième partie d'élément d'appui (57) en raison de la force de fixation.

6. Système de fixation selon la revendication 5, **caractérisé par**
a) une limite de partie discrète en termes de déformabilité de l'élément d'appui (22) entre la première partie d'élément d'appui (55) et la deuxième partie d'élément d'appui (57),
dans lequel, en particulier, la limite de partie est orientée transversalement et en particulier perpendiculairement à une direction de fixation de l'élément de fixation (44) en direction de la partie d'ancrage (24),
ou
b) une partie de transition existante en ce qui concerne la déformabilité de l'élément d'appui (22), par l'intermédiaire de laquelle la première partie d'élément d'appui (55) et la deuxième partie d'élément d'appui (57) se fondent l'une dans l'autre.

7. Système de fixation selon l'une des revendications précédentes, **caractérisé en ce que** l'au moins une zone de déformation (58) présente une extension parallèle à une zone d'appui (54) de l'élément d'appui (22) pour l'élément de stabilisation (16).

8. Système de fixation selon l'une des revendications précédentes, **caractérisé en ce que** l'au moins une zone de déformation (58) est agencée ou formée symétriquement sur l'élément d'appui (22) par rapport à un plan de symétrie (60) contenant un axe (34) de la partie de réception et/ou de l'élément d'appui (22).

9. Système de fixation selon la revendication 8, **caractérisé en ce que** l'évidement de matière
a) est un évidement sur une surface de l'élément d'appui (22), l'élément d'appui (22) étant déformable au bord de l'évidement,
ou
b) est ou comprend une ouverture de passage (62) de l'élément d'appui (22),
l'ouverture de passage (62) présentant, en particulier, une partie transversale circulaire, elliptique, ovale, ronde, en forme de trou oblong ou non circulaire,
ou
c) est une cavité formée dans l'élément d'appui (22) et fermée de tous côtés,
la cavité présentant, en particulier, une partie transversale circulaire, elliptique, ovale, ronde, en forme de trou oblong ou non circulaire.

10. Système de fixation selon la revendication 8 ou 9, **caractérisé en ce que** l'évidement de matière
a) s'étend le long de l'élément de stabilisation (16) agencé dans la partie de réception (26)
et/ou
b) s'étend radialement par rapport à un axe (48) défini par l'élément d'appui (22).

11. Système de fixation selon l'une des revendications précédentes, **caractérisé en ce que** l'élément d'appui (22) comprend deux ou plusieurs parties de déformation (72, 74, 76) qui comprennent des matériaux différents en termes de déformabilité ou sont fabriquées à partir de matériaux différents, l'au moins une zone de déformation (58) étant formée en raison de la nature matérielle différente des deux ou plusieurs parties de déformation (72, 74, 76),
dans lequel, en particulier,
a) les deux ou plusieurs parties de déformation (72, 74, 76) sont des parties de déformation de l'élément d'appui (22) formées séparément les unes des autres et assemblées les unes aux autres, dans lequel, en particulier, une première partie de déformation (72) est prévue, qui est au moins partiellement entourée par au moins une deuxième partie de déformation (74), la déformabilité de la première partie de déformation (72) étant supérieure à la déformabilité de l'au moins une deuxième partie de déformation (74),
et/ou
b) trois parties de déformation (72, 74, 76) ou plus sont prévues, la déformabilité d'une partie déformable respective (72, 74, 76), qui entoure au moins partiellement une autre partie déformable (72, 74, 76), étant inférieure à la déformabilité de la partie de déformation entourée (72, 74, 76).

12. Système de fixation selon l'une des revendications précédentes, **caractérisé en ce que** l'élément d'appui (22) comprend une pluralité de zones de déformation (58), en particulier avec une pluralité d'évidements de matière,
dans lequel, en particulier,
a) deux ou plusieurs zones de déformation (58) de conception identique sont prévues et/ou dans lequel deux ou plusieurs zones de déformation (58) de conception différente sont prévues
et/ou
b) deux zones de déformation (58) sont prévues, qui sont agencées à distance l'une de l'autre et alignées l'une avec l'autre sur l'élément d'appui (22).

13. Système de fixation selon l'une des revendications précédentes, **caractérisé en ce que** l'élément d'appui (22)
a) présente deux zones de déformation (58) diamétralement opposées l'une à l'autre
et/ou
b) comprend deux éléments de support (66) espacés l'un de l'autre, qui délimitent latéralement un renfoncement (70) effilé, l'élément de stabilisation (16) pouvant être positionné entre les éléments de support (66) dans le renfoncement (70), une zone de déformation (58) étant de préférence agencée sur au moins un élément de support (66),
et/ou
c) peut être aligné ou orientable de manière coaxiale par rapport à la partie de réception (26) et/ou à la partie d'ancrage (24).

14. Système de fixation selon l'une des revendications précédentes, **caractérisé en ce que** la partie d'ancrage (24) et l'élément d'appui (22) comprennent des zones d'appui (30, 56) adaptées l'une à l'autre, en particulier en forme de coques sphériques au moins par parties.

15. Système de fixation selon l'une des revendications précédentes, **caractérisé en ce que**
a) le système de fixation comprend au moins l'un des éléments suivants :
- deux ou plusieurs éléments d'ancrage (12) ;
- deux ou plusieurs éléments d'appui (22) ;
- au moins un élément de stabilisation (16), en particulier une pluralité d'éléments de stabilisation (16) ;
- au moins un élément de fixation (44), en particulier une pluralité d'éléments de fixation (44) ;
et/ou
b) au moins l'une des conditions suivantes s'applique :
- l'au moins un élément d'ancrage (12) est une vis à os (14) ;
- l'au moins un élément de stabilisation (16) est un élément de tige (18) ;
l'au moins un élément de fixation (44) est un élément à vis (46) pouvant être vissé sur la partie de réception (26).
